# EUROPÄISCHE PATENTANMELDUNG

(11) **EP 2 407 130 A1**
(43) Veröffentlichungstag der Anmeldung: **18.01.2012**
(21) Anmeldenummer: 10169275.4
(22) Anmeldetag: 12.07.2010
(51) Int. Cl.: A61F 5/56

(54) **Gerät zur Ausgabe von Audiosignalen zum Beeinflussen eines Schlafenden zum Zwecke einer Unterbrechung oder Verringerung von Schnarch-Geräuschen**

(71) Anmelder: Piotrowski, Walter, 68519 Viernheim (DE)
(72) Erfinder: Piotrowski, Walter, 68519 Viernheim (DE)
(74) Vertreter: Zenz

(57) **Zusammenfassung**

Ein Gerät zur Ausgabe von Audiosignalen zum Beeinflussen eines Schlafenden zum Zwecke einer Verringerung von Schnarch-Geräuschen weist eine Audioempfangseinrichtung (1,2), eine Auswerteeinrichtung und eine Ausgabeeinrichtung (10,15) auf. Die Auswerteeinrichtung umfasst eine Verstärkerschaltung (4), eine Schaltung (5) und eine Vergleichsschaltung (6,7,8) wobei eine Steuerschaltung (10) in Abhängigkeit von diesen Vergleichen einerseits die Verstärkung der Verstärkerschaltung (4) so einstellt, dass der Ruhepegel des Hüllsignals in einem vorgegebenen Bereich liegt, und andererseits ein Geräuschanzeigesignal erzeugt, wenn die Vergleichsschaltung ein Überschreiten eines in einem vorgegebenen Abstand über dem vorgebenen Bereich liegenden Schwellwerts durch das Hüllsignal feststellt. Die Steuerschaltung (10) erzeugt das das Schnarch-Geräusch anzeigende Signal, wenn das Geräuschanzeigesignal mit jeweils einer in einem vorgegebenen Bereich liegenden Dauer und mit einer vorgegebenen Anzahl von Wiederholungen innerhalb vorgegebener Zeitintervalle auftrat.

## Beschreibung

Die Erfindung bezieht sich auf ein Gerät zur Ausgabe von Audiosignalen zum Beeinflussen eines Schlafenden zum Zwecke einer Unterbrechung oder Verringerung von Schnarch-Geräuschen mit einer Audioempfangseinrichtung zur Aufnahme von Geräuschen, einer mit der Audioempfangseinrichtung gekoppelten Auswerteeinrichtung zum Erzeugen eines das Schnarch-Geräusch anzeigenden Signals und einer mit der Auswerteeinrichtung gekoppelten Ausgabeeinrichtung, die bei Empfang des das Schnarch-Geräusch anzeigenden Signals Audiosignale erzeugt und über eine Schallerzeugungseinrichtung an den Schlafenden ausgibt.

Ein derartiges Gerät ist beispielsweise aus der Offenlegungsschrift DE 34 39 329 A1 bekannt. Bei diesem Gerät werden die mit Hilfe eines Mikrofons aufgenommenen Geräusche in der Umgebung des Schlafenden zunächst verstärkt und dann in einem Schwellwertschalter-IC mit einem Schwellwert, welcher über ein Potentiometer einstellbar ist, verglichen. Bei Überschreiten des Schwellwerts wird ein Signal erzeugt, welches eine Ausgabeschaltung veranlasst, einen für den Schlafenden unangenehmen Ton über einen Lautsprecher auszugeben. Nachteilig bei diesem bekannten Gerät ist nicht nur, dass es auch auf Geräusche anspricht, welche keine Schnarch-Geräusche darstellen, sondern dass es zudem den Schlaf des Schnarchers in inakzeptabler Weise stört. In der Druckschrift WO 2007/010332 A1 wird vorgeschlagen, die Schlafqualität des Schnarchers dadurch wesentlich zu verbessern, dass das Gerät nach Identifikation der Schnarchgeräusche ein Tonsignal aussendet, dass den Geräuschen von Lippenschmatzen oder Zungenschmatzen ähnelt. Dadurch werde gezielt nur die orofaciale Muskulatur zu einem optimalen Muskeltonus stimuliert, was das sofortige Aussetzen des Schnarchens bewirke.

Ein verbessertes Gerät wird in der Offenlegungsschrift DE 10 2008 000 518 A1 vorgeschlagen. Zum Erkennen der Schnarchgeräusche werden die über ein Mikrofon aufgenommenen und durch einen Verstärker verstärkten Audiosignale nicht nur mit einem Schwellwertpegel verglichen, sondern auch innerhalb einer Verzögerungszeitspanne zunächst ignoriert. Auf diese Weise lassen sich kurzzeitige oder vergleichsweise niedrige Geräuschpegel von den zu überwachenden Schnarchgeräuschen unterscheiden. Die Druckschrift schlägt ferner vor, bei Erfassen eines Schnarch-Geräuschs eine Wiedergabeeinheit zu aktivieren, die dann dafür sorgt, dass eine vorab aufgezeichnete Suggestions-Audiosequenz, insbesondere eine Folge von gesprochenen oder geflüsterten Suggestionen in Verbindung mit einer geeigneten Entspannungsmusik, über eine Schallerzeugungseinrichtung ausgegeben werden.

Nachteilig bei den bekannten Geräten ist insbesondere die unzureichende Erkennung von Schnarch-Geräuschen und deren Unterscheidung von anderen Geräuschen sowie der nicht unerhebliche Aufwand bei der Einstellung der geeigneten Schwellwerte. Aufgabe der Erfindung ist es daher, diese Nachteile zu vermeiden.

Diese Aufgabe wird erfindungsgemäß durch ein Gerät mit den Merkmalen des Anspruchs 1 gelöst. Das erfindungsgemäße Gerät zur Ausgabe von Audiosignalen zum Beeinflussen eines Schlafenden zum Zwecke einer Unterbrechung oder Verringerung von Schnarch-Geräuschen enthält eine Audioempfangseinrichtung zur Aufnahme von Geräuschen und Ausgabe eines entsprechenden Audioeingabesignals, eine mit der Audioempfangseinrichtung gekoppelte Auswerteeinrichtung zum Analysieren des Audioeingabesignals, zum Erkennen von Signaleigenschaften, die ein Schnarch-Geräusch charakterisieren, und zum Erzeugen eines das erkannte Schnarch-Geräusch anzeigenden Signals sowie eine mit der Auswerteeinrichtung gekoppelte Ausgabeeinrichtung, die bei Empfang des das Schnarch-Geräusch anzeigenden Signals Audiosignale erzeugt und über eine Schallerzeugungseinrichtung an den Schlafenden ausgibt. Die Auswerteeinrichtung weist erfindungsgemäß eine Verstärkerschaltung mit einstellbarer Verstärkung auf, die das Audioeingabesignal empfängt und ein eingestelltes Audioeingabesignal erzeugt. Sie weist ferner eine Schaltung auf, die ein der momentanen Amplitude oder dem momentanen Effektivwert des eingestellten Audioeingabesignals entsprechendes Hüllsignal erzeugt, sowie eine Vergleichsschaltung, die das Hüllsignal mit Schwellwerten vergleicht, wobei eine Steuerschaltung in Abhängigkeit von diesen Vergleichen einerseits die Verstärkung der Verstärkerschaltung so einstellt, dass der Ruhepegel des Hüllsignals in einem vorgegebenen Bereich liegt, und andererseits ein Geräuschanzeigesignal erzeugt, wenn die Vergleichsschaltung ein Überschreiten eines in einem vorgegebenen Abstand über dem vorgegebenen Bereich liegenden Schwellwerts durch das Hüllsignal feststellt. Die Steuerschaltung erzeugt dass das Schnarch-Geräusch anzeigende Signal, wenn das Geräuschanzeigesignal mit jeweils einer in einem vorgegebenen Bereich liegenden Dauer und mit einer vorgegebenen Anzahl von Wiederholungen innerhalb vorgegebener Zeitintervalle auftrat.

Das erfindungsgemäße Gerät erlaubt nicht nur eine sehr sichere Unterscheidung von Schnarch-Geräuschen von anderen Geräuschen. Mit dem erfindungsgemäßen Gerät ist es außerdem möglich, ein sofortiges Ansprechen auf ein vereinzeltes oder ein nur mit einer geringen Anzahl von Wiederholungen aufgetretenes Schnarch-Geräusch zu vermeiden. Zudem gestattet das erfindungsgemäße Gerät eine automatische Nachführung bei geänderten oder sich ändernden Umgebungsgeräuschen und vermeidet so eine aufwändige Justage oder Nach-Justage. Diese genannten Vorteile vereint das Gerät mit der Möglichkeit einer relativ einfachen Schaltungsgestaltung. Das Gerät lässt sich mit den üblichen Audiosignalverstärkern, wenigen Komparatoren und einem Mikrocontroller mit zugehörigen Ein- und Ausgabeeinrichtungen preiswert ausführen.

Vorzugsweise ist das Gerät dadurch gekennzeichnet, dass die das Hüllsignal erzeugende Schaltung eine einen Kondensator aufladende Spitzenwertgleichrichterschaltung aufweist, wobei die Zeitkonstante der Entladung des Kondensators einer Frequenz entspricht, die geringer als die tiefste auszuwertende Frequenz des Audioeingabesignals und höher als die höchste zu erwartende Wiederholfrequenz der Schnarch-Geräusche ist. Üblicherweise liegt die tiefste auszuwertende Frequenz des Audiosignals deutlich über 20 Hz und liegt die höchste zu erwartende Wiederholfrequenz der Schnarch-Geräusche deutlich unter 1 Hz, so dass eine schaltungstechnisch relativ einfache Einstellung der Zeitkonstante möglich ist.

Eine bevorzugte Ausführungsform des Geräts ist dadurch gekennzeichnet, dass die Vergleichsschaltung drei Komparatoren umfasst, die das Hüllsignal mit einem kleinsten, einem mittleren bzw. einem größten Schwellwert vergleichen. Die Ausgänge der Komparatoren sind mit Eingängen der Steuerschaltung gekoppelt, wobei die Steuerschaltung einerseits die Verstärkung so einstellt, dass der Ruhepegel des Hüllsignals zwischen dem kleinsten und dem mittleren Schwellwert liegt, und andererseits das Geräuschanzeigesignal ausgibt, wenn das Hüllsignal den größten Schwellwert überschreitet. Vorzugsweise sorgt die Steuerschaltung für eine Erhöhung der eingestellten Verstärkung der Verstärkerschaltung, wenn das Hüllsignal den kleinsten Schwellwert unterschreitet. Sie sorgt für eine Verringerung der Verstärkung, wenn das Hüllsignal auch in den Pausen zwischen den Schnarch-Impulsen, also längerzeitig, den mittleren Schwellwert überschreitet. Die Steuerschaltung sorgt im Grunde dafür, dass das Hüllsignal dann, wenn kein Geräusch-Ereignis auftritt, wenn es somit seinen Ruhepegel aufweist, zwischen dem kleinsten und dem mittleren Schwellwert liegt. Der größte Schwellwert liegt über dem mittleren Schwellwert. Wenn die Steuerschaltung aufgrund des Ausgabesignals des dritten Komparators feststellt, dass das Hüllsignal den größten Schwellwert überschritten hat, wird dies zunächst als Geräuschanzeige, beispielsweise aufgrund eines Schnarch-Geräuschs, interpretiert. Je geringer der Abstand zwischen dem größten und dem mittleren Schwellwert, desto größer ist die Empfindlichkeit dieser Erfassung. Die Verwendung der drei Komparatoren mit den drei abgestuften Schwellwerten erlaubt eine relativ einfache Schaltungsgestaltung. Beispielsweise können die drei Schwellwerte durch einen Widerstandsspannungsteiler mit vier Widerständen realisiert werden, wobei die Abgriffe zwischen den Widerständen des Spannungsteilers mit den nicht-invertierenden Eingängen von drei Operationsverstärkern gekoppelt sind und wobei das Hüllsignal mit den invertierenden Eingängen der drei Operationsverstärker gekoppelt wird.

Bei einer bevorzugten Weiterbildung der zuletzt genannten Ausführungsform ist die Steuerschaltung ein Mikrocontroller mit drei digitalen Eingabeports, die mit den Ausgängen der Komparatoren gekoppelt sind. Der Mikrocontroller weist ferner einen Analogausgabeport auf, der eine Steuerspannung zur Einstellung der Verstärkung der Verstärkerschaltung ausgibt. Im Inneren des Mikrocontrollers ist dem Analogausgabeport üblicherweise ein Digital-Analog-Umsetzer vorgeschaltet.

Bei einer bevorzugten Ausführungsform des Geräts ist die Zeitkonstante, mit der die Steuerschaltung die Verstärkung einstellt, größer als die größte zu erwartende Anstiegszeit des Hüllsignals bei Auftreten eines Schnarch-Geräuschs.

Bei einer bevorzugten Weiterbildung erzeugt die Steuerschaltung dass das Schnarch-Geräusch anzeigende Signal dann, wenn das Geräuschanzeigesignal mit jeweils einer in einem vorgegebenen Bereich liegenden Dauer mehrfach hintereinander mit in einem vorgegebenen Bereich liegenden regelmäßigen zeitlichen Abständen auftrat. Der vorgegebene Bereich für die Dauer und der vorgegebene Bereich für die regelmäßigen zeitlichen Abstände werden jeweils so festgelegt, dass übliche Variationen bei Wiederholungen von Schnarch-Geräuschen berücksichtigt werden. Vorzugsweise speichert die Steuerschaltung einen der Dauer des Anliegens des Geräuschanzeigesignals entsprechenden Wert und einen der Pausenzeit bis zum erneuten Anlegen des Geräuschanzeigesignals entsprechenden Wert. Bei jedem erneuten Anlegen des Geräuschanzeigesignals werden dann die Werte mit den Werten des vorhergehenden Anliegens des Geräuschanzeigesignals verglichen, wobei dann, wenn diese übereinstimmen oder nicht mehr als ein vorgegebenes Toleranzmaß voneinander abweichen, ein Schnarchzykluszähler inkrementiert wird. Weichen sie mehr als das vorgegebene Toleranzmaß voneinander ab, so wird der Schnarchzykluszähler zurückgesetzt (beispielsweise auf Null oder Eins). Vorzugsweise vergleicht die Steuerschaltung den Wert des Schnarchzykluszählers mit einem der vorgegebenen Anzahl von Wiederholungen entsprechenden Wert, wobei sie bei Übereinstimmung das das Schnarch-Geräusch anzeigende Signal erzeugt.

Bei einer bevorzugten Ausführungsform ist die vorgegebene Anzahl von Wiederholungen durch einen Benutzer einstellbar. Beispielsweise kann der Benutzer einstellen, dass die Schnarch-Erkennung erst dann anspricht, wenn drei, vier oder mehr Wiederholungen eines erfassten Schnarch-Geräuschs innerhalb kurzer Zeit hintereinander aufgetreten sind. Selbstverständlich werden nur solche Wiederholungen gezählt, die innerhalb vorgegebener Zeiten hintereinander auftreten. Bleibt für längere Zeit das Schnarch-Geräusch aus, so werden die Zähler vorzugsweise auf Null zurückgesetzt.

Eine bevorzugte Weiterbildung des erfindungsgemäßen Geräts ist dadurch gekennzeichnet, dass die Auswerteeinrichtung eine mit dem Ausgang der Verstärkerschaltung mit einstellbarer Verstärkung gekoppelte Impulserzeugungsschaltung aufweist, die das eingestellte Audioeingabesignal empfängt und eine der Grundfrequenz des eingestellten Audioeingabesignals entsprechende Impulsfolge erzeugt und an einen Eingang der Steuerschaltung anlegt. Die Steuerschaltung zählt die angelegten Impulse während derjenigen Zeitintervalle, in denen das Geräuschanzeigesignal anliegt. Die Steuerschaltung erzeugt dass das Schnarch-Geräusch anzeigende Signal nur dann, wenn neben den oben genannten Bedingungen die Anzahl der während des Anliegens des Geräuschanzeigesignals jeweils gezählten Impulse bei der vorgegebenen Anzahl von Wiederholungen nicht mehr als ein vorgegebenes Maß voneinander abweichen. Auf diese Weise werden nur solche plötzlichen Geräusch-Ereignisse als Schnarch-Geräusch-Ereignisse interpretiert, die von vergleichbarer Dauer sind und zudem ein Geräusch enthalten, welches eine vergleichbare Grundfrequenz aufweist. Dieser Weiterbildung liegt die Erkenntnis zugrunde, dass sich wiederholende Schnarchgeräusche üblicherweise in vergleichbaren Frequenzbereichen auftreten. Somit werden nicht nur Dauer und Abstand von Geräusch-Ereignissen zur Schnarch-Erkennung herangezogen, sondern außerdem die Grundfrequenzen der aufeinanderfolgenden Ereignisse miteinander verglichen, wodurch eine Art Plausibilitätsprüfung stattfindet.

Eine bevorzugte Weiterbildung der zuletzt genannten Ausführungsform ist dadurch gekennzeichnet, dass die Steuerschaltung das das Schnarch-Geräusch anzeigende Signal nur dann erzeugt, wenn das Geräuschanzeigesignal mehrfach mit der vorgegebenen Anzahl hintereinander in näherungsweise gleichen Abständen mit näherungsweise gleicher Dauer anlag und dabei die Anzahlen der während der Anliegens gezählten Impulse näherungsweise gleich blieben. Als näherungsweise gleich bewertet werden Abstände, Dauern bzw. Anzahlen, die um nicht mehr als ein vorgegebenes Toleranzmaß voneinander abweichen. Vorzugsweise liegen die vorgegebenen Toleranzmaße für Abstände, Dauern bzw. Anzahlen zwischen 50 % und 10 %.

Vorzugweise umfasst die Impulserzeugungsschaltung einen als Nulldurchgangsdetektor des eingestellten Audioeingabesignals betriebenen Komparator. Dies vereinfacht die Schaltung. Dieser Komparator weist vorzugsweise eine geringe Hysterese auf, um eine unerwünschte Impulserzeugung bei sehr geringen Pegelschwankungen des eingestellten Audioeingabesignals um den Nullpunkt herum zu vermeiden.

Eine bevorzugte Weiterbildung des Geräts ist dadurch gekennzeichnet, dass die die Audioausgabesignale erzeugende Ausgabeeinrichtung eine Audiosequenz ausgibt, welche neben einer ausgewählten Hintergrundmusik und/oder ausgewählten Hintergrundgeräuschen gesprochene suggestive Anweisungen an den Schlafenden enthält. Vorzugsweise ist die Audiosequenz als Datei (oder auch in Form mehrerer Dateien) in einem digitalen Format gespeichert, wobei die Ausgabeeinrichtung die Datei liest und in Audiosignale konvertiert.

Vorteilhafte und/oder bevorzugte Weiterbildungen der Erfindung sind in den Unteransprüchen gekennzeichnet.

Nachfolgend wird die Erfindung anhand eines in den Zeichnungen dargestellten bevorzugten Ausführungsbeispiels näher erläutert, wobei in den Zeichnungen:
Figur 1 ein Blockschaltbild eines bevorzugten Ausführungsbeispiels des erfindungsgemäßen Geräts zeigt; und
Figur 2 Signalverläufe ausgewählter Signale innerhalb des Geräts gemäß Figur 1 veranschaulicht.

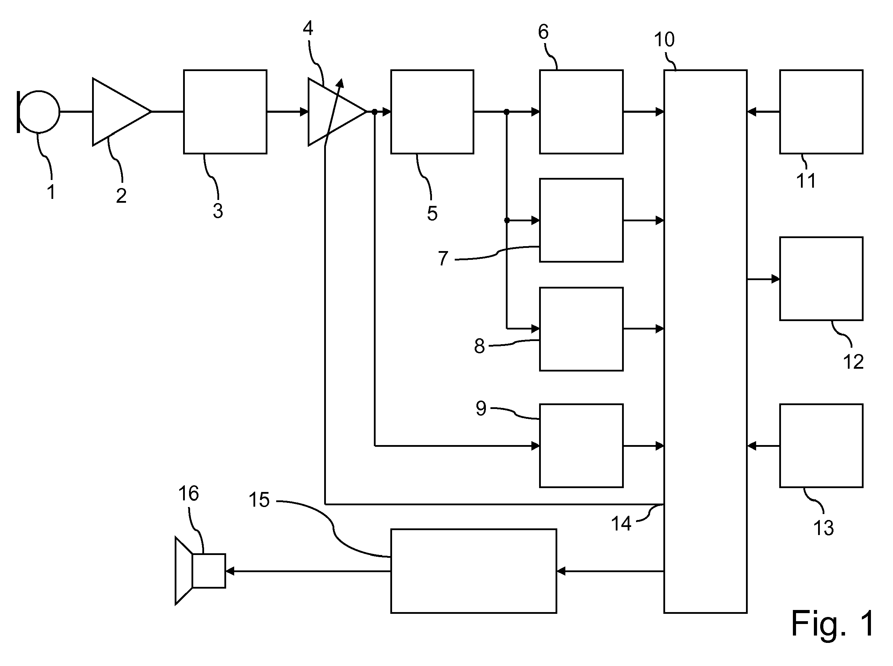

Figur 1 zeigt eine schematische Blockdarstellung eines Ausführungsbeispiels des erfindungsgemäßen Geräts, welches der Ausgabe von Audiosignalen zum Beeinflussen eines Schlafenden zum Zwecke einer Unterbrechung oder Verringerung von Schnarch-Geräuschen dient. Das Gerät weist als Audioempfangseinrichtung ein Mikrofon 1 auf, welches mit dem Eingang eines Vorverstärkers 2 gekoppelt ist. Das Mikrofon ist beispielsweise ein empfindliches Elektret-Mikrofon. Der Vorverstärker ist beispielsweise unter Verwendung eines Operationsverstärkers realisiert. Der Ausgang des Vorverstärkers 2 ist mit einem Tiefpass-Filter 3 gekoppelt, welches eine Grenzfrequenz von beispielsweise 4 kHz aufweist. Mittels dieses Tiefpass-Filters 3 werden die für die Schnarch-Erkennung nicht maßgeblichen Frequenzanteile herausgefiltert. Das gefilterte Audioeingabesignal gelangt dann zu einem zweiten Verstärker 4, dessen Verstärkung über eine Steuerspannung einstellbar ist. Am Ausgang des Verstärkers 4 liegt das in seinem Pegel eingestellte Audioeingabesignale vor. Das eingestellte Audioeingabesignal wird dann einer Spitzenwertgleichrichterschaltung 5 zugeführt, die einen Kondensator auflädt, wobei die durch den Lastwiderstand bestimmte Entladezeitkonstante so gewählt ist, dass sich am Ausgang der Spitzenwertgleichrichterschaltung ein analoges Hüllsignal ergibt, dessen Augenblickswert der momentanen Amplitude oder dem momentanen Effektivwert des eingestellten Audioeingabesignals entspricht, vorzugsweise proportional ist.

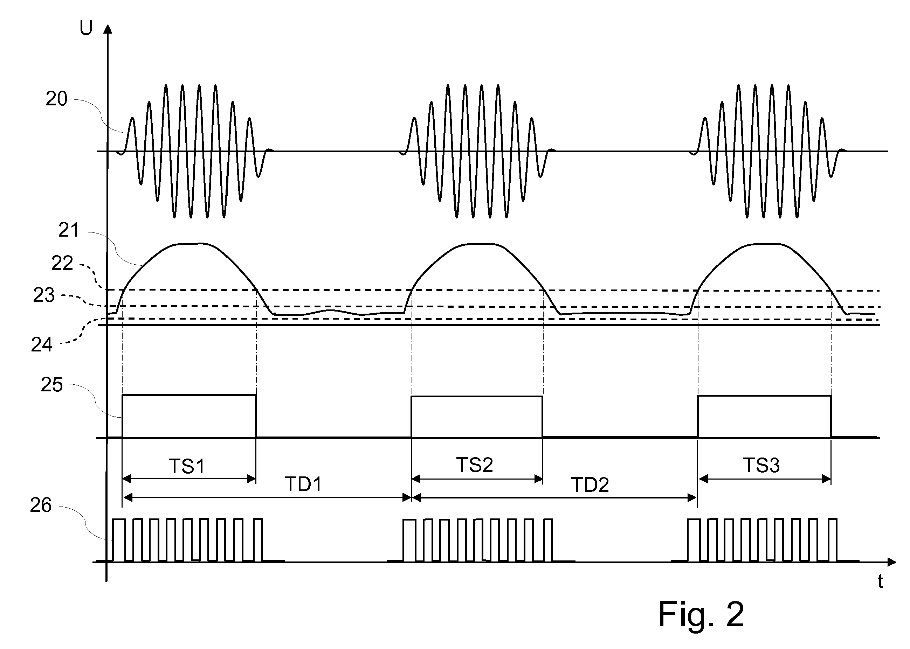

Figur 2 veranschaulicht einige Signalverläufe von in der Schaltung gemäß Figur 1 auftretenden Signalen. Der in Figur 2 oben gezeigte Signalverlauf 20 stellt das eingestellte Audioeingabesignal am Ausgang des Verstärkers 4 dar. Schematisch sind hier Signalverläufe von drei in bestimmten Abständen auftretenden Schnarch-Geräuschen veranschaulicht. Der Signalverlauf 20 ist gegenüber den tatsächlichen Signalverläufen stark vereinfacht; bei den tatsächlichen Audiosignalen entstehen kompliziertere Verläufe aufgrund des Frequenzgemisches der Geräusche. Bei dem vereinfachten Signalverlauf 20 ist insbesondere eine Grundfrequenz erkennbar, die auch bei jedem realen Schnarch-Ereignis vorhanden ist. Der darunter gezeigte Signalverlauf 21 stellt das

Ausgangssignal der Spitzenwertgleichrichterschaltung 5, also das Hüllsignal, dar. Man erkennt, dass das Ausgangssignal der Spitzenwertgleichrichterschaltung 5 ausgehend von einem Ruhepegel bei jedem Schnarch-Ereignis zu einem impulsartigen Spannungsverlauf führt.

Es wird wieder auf Figur 1 Bezug genommen. Der Ausgang der Spitzenwertgleichrichterschaltung 5 ist mit den Eingängen von drei Komparatorschaltungen 6, 7 und 8 verbunden. Jede der drei Komparatorschaltungen 6, 7 und 8 vergleicht den Augenblickswert des Hüllsignals mit einem vorgegebenen Schwellwert. Die drei Schwellwerte sind in Figur 2 durch drei gestrichelte Linien 22, 23 und 24 veranschaulicht. Der Komparator 6 vergleicht den Signalverlauf 21 des Hüllsignals mit dem niedrigsten Schwellwert 24, der Komparator 7 vergleicht das Hüllsignal 21 mit dem mittleren Schwellwert 23, und der Komparator 8 vergleicht das Hüllsignal 21 mit dem höchsten Schwellwert 22. Die Ergebnisse der Vergleiche entsprechen den Ausgangssignalen der Komparatoren 6, 7 und 8. Sofern der Pegel des Hüllsignals 21 über dem jeweiligen Schwellwert 22, 23 bzw. 24 liegt, wird ein hoher Pegel abgegeben, anderenfalls ein niedriger Pegel. Die Ausgänge der Komparatoren 6, 7 und 8 sind mit digitalen Eingabeports eines Mikrocontrollers 10 gekoppelt.

Der Mikrocontroller 10 dient der Steuerung des gesamten Geräts. In Abhängigkeit von den Ausgabesignalen der Komparatoren 6, 7 und 8 steuert der Mikrocontroller 10 einerseits die Verstärkung des Verstärkers 4 und andererseits die Ausgabe von Audiosignalen zum Beeinflussen eines Schlafenden zum Zwecke einer Unterbrechung oder Verringerung von Schnarch-Geräuschen.

Zunächst sorgt der Mikrocontroller 10 in Abhängigkeit von den Ausgabesignalen der Komparatoren mit dem niedrigsten und dem mittleren Schwellwert für eine Einstellung der Verstärkung des Verstärkers 4 derart, dass der Ruhepegel des eingestellten Audioeingabesignals bzw. der Ruhepegels des Hüllsignals immer zwischen dem niedrigsten Schwellwert 24 und dem mittleren Schwellwert 23 verbleibt. Sinkt der Pegel des Hüllsignals 21 unter den niedrigsten Schwellwert 24 ab, so erhöht der Mikrocontroller 10 die Verstärkung des Verstärkers 4, indem er die über einen analogen Ausgabeport 14 ausgegebene Steuerspannung erhöht. Steigt der Pegel des Hüllsignals 21 während der Ruhezeiten über den mittleren Schwellwert 23, so verringert der Mikrocontroller 10 die Verstärkung des Verstärkers 4, indem er die über den Ausgabeport 14 ausgegebene Steuerspannung wieder absenkt. Durch diese automatische Anpassung an den Grundgeräuschpegel passt sich das Gerät automatisch an verschiedene und auch während der Nacht wechselnde Umgebungsbedingungen an. Wenn beispielsweise in den Morgenstunden die von außen in die Schlafräume eindringenden Geräusche zu einem erhöhten Grundrauschen führen, so wird durch die automatische Einstellung der Verstärkung erreicht, dass Fehlerkennungen von Schnarch-Geräuschen vermieden werden. Der unterste Schwellenwert 24 und der mittlere Schwellenwert 23 haben einen geringen Abstand zueinander und weisen auch absolut relativ geringe Werte auf, da sie lediglich zur Anpassung an den Ruhepegel dienen. Die Auswertung durch den Mikrocontroller 10 ist derart programmiert, dass das Hüllsignal den mittleren Schwellwert 23 kurzfristig überschreiten darf, bevor die Verstärkung dann zurückgeregelt wird. Dies ist insbesondere deshalb erforderlich, weil eine Verstärkungsänderung durch kurzzeitig auftretende Schnarch-Ereignisse zu vermeiden ist.

Der Ausgang des Komparators 8, der das Überschreiten des höchsten Schwellwerts 22 überwacht, wird von dem Mikrocontroller derart ausgewertet, dass bei Überschreiten des höchsten Schwellwerts 22 ein Signal erzeugt wird (indem beispielsweise in dem Mikrocontroller 10 ein Bit gesetzt wird), welches ein Geräusch-Ereignis anzeigt, bei dem es sich um ein Schnarch-Geräusch handeln kann. Der Mikrocontroller 10 überwacht dann programmgesteuert einerseits die Dauer des Anliegens des Ausgangssignals des Komparators 8, also die Dauer des Überschreitens des höchsten Schwellwerts 22, als auch den Abstand zwischen den einzelnen Geräusch-Ereignissen, in denen dieser Schwellwert überschritten wird. Das Ausgangssignal des Komparators 8, welches dem Geräuschanzeigesignal in dem Mikrocontroller (beispielsweise einem gesetzten Bit) entspricht, ist in Figur 2 durch den Verlauf 25 angezeigt. Die dünne Strich-Punkt-Linie zwischen den Orten, in denen der Verlauf 21 den Schwellwert 22 über- bzw. unterschreitet, kennzeichnet das Anlagen und Wegnehmen de Geräuschanzeigesignals aufgrund dieser Schwellwertüber- bzw. -unterschreitung. Die jeweilige Dauer des Anliegens des Geräuschanzeigesignals ist durch "TS1" bis TS3" in Figur 2 gekennzeichnet. Die Zeitintervalle "TD1" und "TD2" kennzeichnen die Zeiten zwischen dem Anlegen des Geräuschanzeigesignals bei einem Ereignis und dem Anlegen des Geräuschanzeigesignals bei dem nachfolgenden Ereignis. Die Differenz TDn-TSn, also beispielsweise TD1-TS1, kennzeichnet dann den Abstand zwischen den Geräuschereignissen. Der Mikrocontroller 10 erfasst programmgesteuert sowohl die Zeiten TSn als auch die Zeiten TDn. In Abhängigkeit von den ermitteln Zeiten, welche in Arbeitsspeichern abgelegt werden, bestimmt der Mikrocontroller, ob es sich um eine Geräusch-Sequenz handelt, welche durch Schnarchen verursacht wurde. Sofern die Zeiten des Anliegens des Geräuschanzeigesignals TS innerhalb eines vorgegebenen Intervalls liegen und die aufeinanderfolgenden Zeiten eine vergleichbare Dauer aufweisen, das heißt maximal um ein vorgegebenes Toleranzmaß voneinander abweichen, und sofern diese Geräuschanzeigesignale mit gleichmäßigen Abständen angelegt werden, so werden die einzelnen Ereignisse als Schnarch-Ereignisse bewertet. Diese führt dazu, dass bei jedem Schnarch-Ereignis ein Zähler, Schnarchzykluszähler genannt, um 1 inkrementiert wird. Sofern der Schnarchzykluszähler einen vorgegebenen, vom Bediener einstellbaren Wert erreicht, der beispielsweise zwischen 2 und 9 liegt, erzeugt der Mikrocontroller 10 ein Signal (beispielsweise in Form einer Ausgabevariable oder eines gesetzten Bits), welches das Schnarch-Geräusch anzeigt. Dieses Signal wird beispielsweise auf einer mit dem Mikrocontroller 10 gekoppelten Ausgabeeinrichtung 12 angezeigt. Vor allem aber führt das Anlegen dieses das Schnarch-Geräusch anzeigenden Signals dazu, dass aus einem Speicher 13 eine in Form einer Datei abgespeicherte digitale Audiosequenz abgerufen und einer Wiedergabeschaltung 15 zugeführt wird. Vorzugsweise wird eine im MP3-Format vorliegende Datei einer Schaltung 15 zugeführt, die einen MP3-Dekodierer enthält. Die Schaltung 15 sorgt für eine Wiedergabe dieser Datei über einen Lautsprecher 16. Die MP3-Datei oder eine Audiosequenz in einem anderen Datenformat führt beispielsweise zur Wiedergabe einer Hintergrundmusik und zusätzlicher Geräusche (beispielsweise Meeresrauschen) in Kombination mit suggestiven Anweisungen an den Schlafenden. Die Auswahl verschiedener MP3-Dateien kann mit Hilfe einer mit dem Mikrocontroller 10 gekoppelten Eingabeeinrichtung 11, unterstützt durch die Anzeigeeinrichtung 12, durch den Bediener vorgenommen werden und dabei an die Bedürfnisse des Schlafenden angepasst werden. Die Datei wird mit einer Lautstärke wiedergegeben, die nicht zu einem Aufwecken des Schlafenden führt. Bei einer bevorzugten Ausführungsform sind beispielsweise vier unterschiedliche Audiosequenzen, die vom Bediener ausgewählt werden können, in der Speichereinrichtung 13, die beispielsweise eine Speicherkarte umfasst, abgelegt. Die Wiedergabedauer beträgt beispielsweise 3 Minuten. Die gesprochenen suggestiven Anweisungen sind derart in die Wiedergabe eingebettet, dass diese von dem Schlafenden befolgt werden. Der Schlafende wird dazu gebracht, sich in die Seitenlage zu drehen und entspannt weiter zu schlafen.

Nachdem der Mikrocontroller 10 für eine Wiedergabe der Audiosequenz, insbesondere der MP3-Datei, gesorgt hat, versetzt er das Gerät wieder in den Grundzustand zurück, wonach erneut die Audioeingabesignale, die von dem Mikrofon 1 aufgenommen wurden, bezüglich möglicher Schnarch-Geräusche ausgewertet werden.

Um die Selektivität bezüglich zu erkennender Schnarch-Geräusche weiter zu erhöhen und die Wahrscheinlichkeit des Reagierens auf andere Geräusche zu verringern, enthält das in Figur 1 dargestellte Gerät eine zusätzliche Impulserzeugungsschaltung 9, deren Eingang ebenfalls mit dem Ausgang des Verstärkers 4, also mit dem eingestellten Audioeingabesignal, gekoppelt ist. Die Impulserzeugungsschaltung enthält vorzugweise einen einfachen Komparator, der die Nulldurchgänge des Audioeingabesignals erfasst. Die Schwelle des Komparators liegt somit bei dem Wert Null, wobei allerdings der Komparator eine geringfügige Hysterese aufweist, um unkontrollierte Signalübergänge bei geringen Pegeln (insbesondere während der Ruhepegel) zu vermeiden. Der in Figur 2 dargestellte unterste Signalverlauf 26 zeigt die am Ausgang der Schaltung 9 anliegenden Impulse. Ein Impuls liegt jeweils an, solange der oben dargestellte Signalverlauf 20 einen positiven Wert einnimmt. Aufgrund der geringfügigen Hysterese werden unregelmäßige Impulse während der Ruhepegelzeiten vermieden oder verringert. Die am Ausgang der Schaltung 9 auftretenden Impulse variieren sowohl hinsichtlich der Impulsbreiten als auch der Impulsabstände. Diese unregelmäßigen Impulse werden einem Eingabeport des Mikrocontrollers 10 eingegeben. Programmgesteuert sorgt der Mikrocontroller 10 nunmehr dafür, dass die Impulse während des Anliegens des Geräuschanzeigesignals, das heißt während des Anliegens des Ausgabesignals des Komparators 8, gezählt werden. Wie in Figur 2 zu erkennen ist, werden die Impulse des Signalverlaufs 26 jeweils während der Zeiten TS1 bis TS3, also während des Anliegens des Signals des Signalverlaufs 25, gezählt. Der Mikrocontroller zählt somit lediglich die mittlere Anzahl von Nulldurchgängen während des jeweiligen Geräuschereignisses. Die variierenden Impulsbreiten und Impulsabstände sind hierbei von untergeordneter Bedeutung. Der Mikrocontroller braucht auch nicht zu ermitteln, ob die Grundfrequenz, welche durch die Zählung der Nulldurchgänge bestimmt werden kann, in einem vorgegebenen, für Schnarch-Geräusche typischen Bereich liegt. Stattdessen ermittelt der Mikrocontroller lediglich, ob bei aufeinanderfolgenden Geräusch-Ereignissen die Anzahl der gezählten Impulse vergleichbar ist, das heißt um nicht mehr als ein vorgegebenes Toleranzmaß voneinander abweicht. Dies vereinfacht die Programmierung des Mikrocontrollers. Hierbei geht die Erfindung von der Erkenntnis aus, dass typischerweise sich wiederholende Schnarch-Geräusche hinsichtlich der Dauer, des Abstands und der mittleren Frequenz vergleichbar sind, das heißt um maximal ein vorgegebenes Toleranzmaß voneinander abweichen.

Vorzugsweise weist das Gerät einen nicht-flüchtigen Speicher zum Speichern der jeweils letzten Einstellungen des Geräts, das heißt der gewünschten Wiedergabe-Lautstärke, der Anzahl der zum Aktivieren erforderlichen Schnarchzyklen und der Auswahl der MP3-Datei, auf. so dass diese Einstellungen auch bei ausgeschaltetem Gerät erhalten bleiben und nach erneutem Einschalten automatisch wieder zur Verfügung stehen.

Bei alternativen Ausführungsformen des erfindungsgemäßen Geräts könnten beispielsweise anstelle der drei Komparatoren 6, 6, 7 und 8 und der Ausgabe der Ausgangssignale der drei Komparatoren an digitale Eingabeports des Mikrocontrollers 10 auch ein analoger Eingabeport des Mikrocontrollers 10 in Verbindung mit einem Analog-Digital-Umsetzer verwendet werden, wobei dann die Abfrage des Überschreitens der Schwellwerte programmtechnisch im Mikrocontroller 10 realisiert wird. Bei einer weiteren alternativen Ausführungsform könnten auch die Schaltung 9 und der separate Spitzenwertgleichrichter 5 entfallen, wobei dann das hinsichtlich der Verstärkung eingestellte Ausgangssignal des Verstärkers 5 direkt einem analogen Eingabeport des Mikrocontrollers 10 zugeführt wird. Der Mikrocontroller würde dann zugleich die Funktionen der das Hüllsignal erzeugenden Schaltung, der Vergleichsschaltung und der Steuerschaltung übernehmen. In diesem Falle läge das Hüllsignal als periodisch aktualisierter Digitalwert im Inneren des Mikrocontrollers vor. Dieser jeweils aktualisierte Wert des Hüllsignals würde periodisch mit den abgespeicherten Schwellwerten verglichen werden. Bei anderen alternativen Ausführungsformen könnte auch ein Teil des bei der bevorzugten Ausführungsform im Mikrocontroller 10 realisierten Funktionen in externen Schaltungen realisiert werden. So könnte beispielsweise der Ausgang der Impulserzeugungsschaltung 9 mit dem Ausgang des Komparators 8 durch ein UND-Gatter verknüpft werde, dessen Ausgang wiederum einem Eingabeport des Mikrocontrollers 10 zugeführt würde.

## Patentansprüche

1. Gerät zur Ausgabe von Audiosignalen zum Beeinflussen eines Schlafenden zum Zwecke einer Unterbrechung oder Verringerung von Schnarch-Geräuschen mit:
einer Audioempfangseinrichtung (1, 2) zur Aufnahme von Geräuschen und Ausgabe eines entsprechenden Audioeingabesignals,
einer mit der Audioempfangseinrichtung (1, 2) gekoppelten Auswerteeinrichtung zum Analysieren des Audioeingabesignals, zum Erkennen von Signaleigenschaften, die ein Schnarch-Geräusch charakterisieren, und zum Erzeugen eines das erkannte Schnarch-Geräusch anzeigenden Signals, und
einer mit der Auswerteeinrichtung gekoppelten Ausgabeeinrichtung (10, 15), die bei Empfang des das Schnarch-Geräusch anzeigenden Signals Audiosignale erzeugt und über eine Schallerzeugungseinrichtung (16) an den Schlafenden ausgibt,
wobei die Auswerteeinrichtung aufweist:
eine Verstärkerschaltung (4) mit einstellbarer Verstärkung, die das Audioeingabesignal empfängt und ein eingestelltes Audioeingabesignal erzeugt,
eine Schaltung (5), die ein der momentanen Amplitude oder dem momentanen Effektivwert des eingestellten Audioeingabesignals entsprechendes Hüllsignal erzeugt,
eine Vergleichsschaltung (6, 7, 8), die das Hüllsignal mit Schwellwerten vergleicht, wobei eine Steuerschaltung (10) in Abhängigkeit von diesen Vergleichen einerseits die Verstärkung der Verstärkerschaltung (4) so einstellt, dass der Ruhepegel des Hüllsignals in einem vorgegebenen Bereich liegt, und andererseits ein Geräuschanzeigesignal erzeugt, wenn die Vergleichsschaltung ein Überschreiten eines in einem vorgegebenen Abstand über dem vorgegebenen Bereich liegenden Schwellwerts durch das Hüllsignal feststellt, und
wobei die Steuerschaltung (10) das das Schnarch-Geräusch anzeigende Signal erzeugt, wenn das Geräuschanzeigesignal mit jeweils einer in einem vorgegebenen Bereich liegenden Dauer und mit einer vorgegebenen Anzahl von Wiederholungen innerhalb vorgegebener Zeitintervalle auftrat.

2. Gerät nach Anspruch 1, **dadurch gekennzeichnet, dass** die das Hüllsignal erzeugende Schaltung (5) eine einen Kondensator aufladende Spitzenwertgleichrichterschaltung aufweist, wobei die Zeitkonstante der Entladung des Kondensators einer Frequenz entspricht, die geringer als die tiefste auszuwertende Frequenz des Audioeingabesignals und höher als die höchste zu erwartende Wiederholfrequenz der Schnarch-Geräusche ist.

3. Gerät nach Anspruch 1 oder 2, **dadurch gekennzeichnet, dass** die Vergleichsschaltung drei Komparatoren (6, 7, 8) umfasst, die das Hüllsignal mit einem kleinsten, einem mittleren bzw. einem größten Schwellwert vergleichen, wobei die Ausgänge der Komparatoren (6, 7, 8) mit Eingängen der Steuerschaltung (10) gekoppelt sind, wobei die Steuerschaltung
die Verstärkung so einstellt, dass der Ruhepegel des Hüllsignals zwischen dem kleinsten und dem mittleren Schwellwert liegt, und
das Geräuschanzeigesignal ausgibt, wenn das Hüllsignal den größten Schwellwert überschreitet.

4. Gerät nach Anspruch 3, **dadurch gekennzeichnet, dass** die Steuerschaltung einen Mikrocontroller (10) mit drei digitalen Eingabeports, die mit den Ausgängen der Komparatoren (6, 7, 8) gekoppelt sind, und mit einem Analogausgabeport (14), der eine Steuerspannung zur Einstellung der Verstärkung der Verstärkerschaltung (4) ausgibt, umfasst.

5. Gerät nach Anspruch 3 oder 4, **dadurch gekennzeichnet, dass** die Zeitkonstante, mit der die Steuerschaltung (10) die Verstärkung einstellt, größer als die größte zu erwartende Anstiegszeit des Hüllsignals bei Auftreten eines Schnarch-Geräuschs ist.

6. Gerät nach einem der Ansprüche 1 - 5, **dadurch gekennzeichnet, dass** die Steuerschaltung (10) das das Schnarch-Geräusch anzeigende Signal erzeugt, wenn das Geräuschanzeigesignal mit jeweils einer in einem vorgegebenen Bereich liegenden Dauer mehrfach hintereinander mit in einem vorgegebenen Bereich liegenden regelmäßigen zeitlichen Abständen auftrat.

7. Gerät nach Anspruch 6, **dadurch gekennzeichnet, dass** die Steuerschaltung (10) einen der Dauer des Anliegens des Geräuschanzeigesignals entsprechenden Wert und einen der Pausenzeit bis zum erneuten Anlegen des Geräuschanzeigesignals entsprechenden Wert speichert und bei jedem erneuten Anlegen des Geräuschanzeigesignals die Werte mit den Werten des vorhergehenden Anliegens des Geräuschanzeigesignals vergleicht und dann, wenn diese übereinstimmen oder nicht mehr als ein vorgegebenes Toleranzmaß voneinander abweichen, einen Schnarchzykluszähler inkrementiert, anderenfalls den Schnarchzykluszähler zurücksetzt.

8. Gerät nach Anspruch 7, **dadurch gekennzeichnet, dass** die Steuerschaltung (10) den Wert des Schnarchzykluszählers mit der vorgegebenen Anzahl von Wiederholungen vergleicht und bei Übereinstimmung das das Schnarch-Geräusch anzeigende Signal erzeugt.

9. Gerät nach einem der Ansprüche 1 - 8, **dadurch gekennzeichnet, dass** die vorgegebene Anzahl von Wiederholungen von einem Benutzer einstellbar ist.

10. Gerät nach einem der Ansprüche 1 - 9, **dadurch gekennzeichnet, dass** die Auswerteeinrichtung eine mit dem Ausgang der Verstärkerschaltung (4) mit einstellbarer Verstärkung gekoppelte Impulserzeugungsschaltung (9) aufweist, die das eingestellte Audioeingabesignal empfängt und eine der Grundfrequenz des eingestellte Audioeingabesignal entsprechende Impulsfolge an einen Eingang der Steuerschaltung (10) anlegt, wobei die Steuerschaltung (10) die angelegten Impulse während derjenigen Zeitintervalle zählt, in denen das Geräuschanzeigesignal anliegt, und wobei die Steuerschaltung (10) das das Schnarch-Geräusch anzeigende Signal nur dann erzeugt, wenn die Anzahlen der während des Anliegens des Geräuschanzeigesignals jeweils gezählten Impulse bei der vorgegebenen Anzahl von Wiederholungen nicht mehr als ein vorgegebenes Maß voneinander abweichen.

11. Gerät nach Anspruch 10, **dadurch gekennzeichnet, dass** die Steuerschaltung (10) das das Schnarch-Geräusch anzeigende Signal nur dann erzeugt, wenn das Geräuschanzeigesignal mehrfach mit der vorgegebenen Anzahl hintereinander in näherungsweise gleichen Abständen mit näherungsweise gleicher Dauer anlag und die Anzahlen der während des Anliegens gezählten Impulse dabei näherungsweise gleich blieben, wobei um bis zu einem jeweils vorgegebenen Toleranzmaß abweichende Abstände, Dauern bzw. Anzahlen als näherungsweise gleich bewertet werden.

12. Gerät nach Anspruch 11, **dadurch gekennzeichnet, dass** die vorgegebenen Toleranzmaße zwischen 50 % und 10 % liegen.

13. Gerät nach einem der Ansprüche 10 - 12, **dadurch gekennzeichnet, dass** die Impulserzeugungsschaltung (9) einen als Nulldurchgangsdetektor des eingestellten Audioeingabesignals betriebenen Komparator umfasst.

14. Gerät nach einem der Ansprüche 1 - 13, **dadurch gekennzeichnet, dass** die die Audioausgabesignale erzeugende Ausgabeeinrichtung (10, 15) eine Audiosequenz ausgibt, welche neben einer ausgewählten Hintergrundmusik und/oder ausgewählten Hintergrundgeräuschen gesprochene suggestive Anweisungen an den Schlafenden enthält.

15. Gerät nach Anspruch 14, **dadurch gekennzeichnet, dass** die Audiosequenz als Datei in einem digitalen Format gespeichert ist, wobei die Ausgabeeinrichtung (10, 15) die Datei liest und in Audiosignale konvertiert.
